# EUROPEAN PATENT APPLICATION

(11) **EP 3 973 777 A1**
(43) Date of publication of application: **30.03.2022**
(21) Application number: 21198599.9
(22) Date of filing: 23.09.2021
(51) Int. Cl.: A01N 25/02, A01N 25/34, A01N 43/16, A41D 19/00, A41D 31/00, A61B 42/00, A01P 1/00

(54) **ANTIMICROBIAL ELASTOMERIC ARTICLE**

(30) Priority: 24.09.2020 MY PI2020004969
(71) Applicant: TOP GLOVE INTERNATIONAL SDN. BHD., 41050 Klang Selangor (MY)
(72) Inventor: WONG, Chong Ban, 41050 KLANG, SELANGOR (MY); MOHAMAD, Nuriah, 41050 KLANG, SELANGOR (MY); P.CHANDRASAKARAN, Devi Shantini, 41050 KLANG, SELANGOR (MY); SELVADURAI, Anuja, 41050 KLANG, SELANGOR (MY); SUBRAMANIAM, Rovethra, 41050 KLANG, SELANGOR (MY)
(74) Representative: Schiweck Weinzierl Koch Patentanwälte Partnerschaft mbB

(57) **Abstract**

The present invention relates to an antimicrobial coagulant solution, wherein the antimicrobial coagulant solution is a mixture of an antimicrobial additive and a coagulant solution. An antimicrobial coagulant solution is a mixture of antimicrobial additive and a coagulant solution, wherein the antimicrobial additive is selected from the group consisting of neem extract or lemon extract and wherein the coagulant solution comprises an antitack agent, a coagulating agent, a wetting agent, a pH adjuster and a solvent.

## Description

### FIELD OF THE INVENTION

The present invention relates to antimicrobial elastomeric articles wherein the antimicrobial elastomeric articles having antimicrobial coating. The antimicrobial coating is prepared from antimicrobial coagulant solution which comprises antimicrobial additive derived from plant.

### BACKGROUND OF THE INVENTION

A primary concern in the healthcare environment is protecting the patient from endogenous, exogenous or nosocomial infections. Elastomeric article, in particular glove has become an element of personal protective equipment and essential part of clinical practice for healthcare workers. It actually safeguards both glove wearer and patient from infection.

Antimicrobial gloves are useful for reducing nosocomial infection by bacteria, fungi and viruses. An unresolved problem for developing antimicrobial gloves is the difficulty of maintaining the antimicrobial efficacy during and after production process. Antimicrobial additive coated on the glove surface is not chemically bound and easily detach from the glove surface. The antimicrobial additive will also degrade during the production process due to high temperature and unable to tolerate with coagulant solution. As a result, the antimicrobial additive on the glove surface is significantly reduced and eventually the antimicrobial efficacy of the glove is also reduced.

To overcome the shortcomings, an approach is developed to produce antimicrobial gloves which are able to tolerate with the coagulant solution and meet the desired antimicrobial efficacy by using antimicrobial additive derived from plant.

### SUMMARY OF THE INVENTION

The present invention relates to an antimicrobial coagulant solution. The antimicrobial coagulant solution is a mixture of an antimicrobial additive and a coagulant solution. The antimicrobial additive is selected from the group consisting of neem extract or lemon extract and wherein the coagulant solution comprises an antitack agent, a coagulating agent, a wetting agent, a pH adjuster and a solvent. Further, present invention discloses antimicrobial elastomeric articles having antimicrobial coating that is prepared from the antimicrobial coagulant solution. The antimicrobial elastomeric article is an antimicrobial glove.

### BRIEF DESCRIPTION OF THE ACCOMPANYING DRAWINGS

The present invention will be fully understood from the detailed description given herein below and the accompanying drawings which are given by way of illustration only, and thus are not limitative of the present invention, wherein:
In the appended drawings:

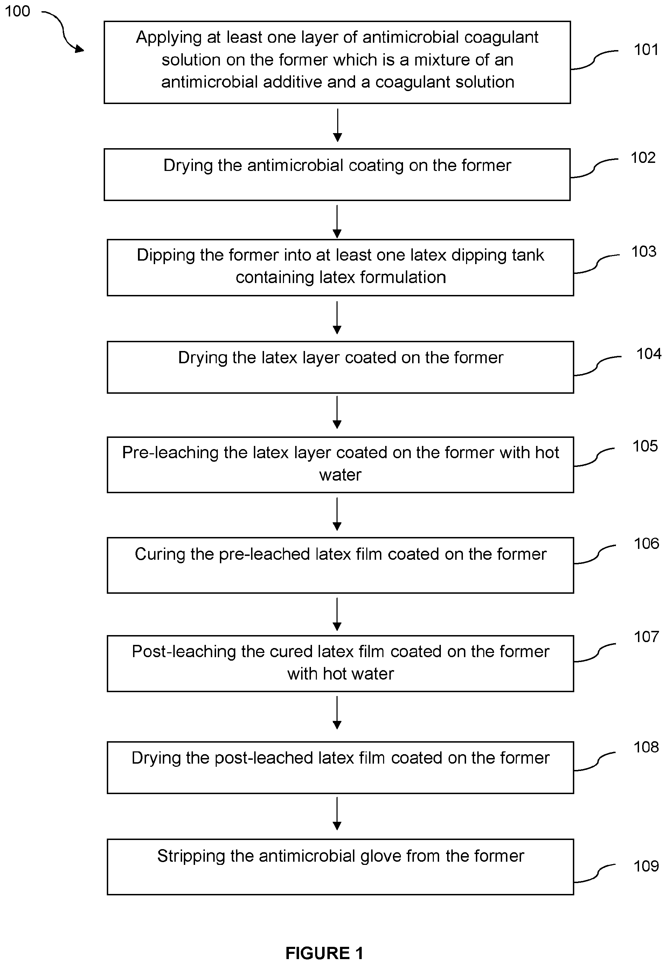

**FIGURE 1** shows a flow diagram of the method for producing antimicrobial gloves in accordance with the exemplary embodiment of the present invention.

### DETAILED DESCRIPTION OF THE INVENTION

Detailed description of preferred embodiments of the present invention is disclosed herein. It should be understood, however, that the embodiments are merely exemplary of the present invention, which may be embodied in various forms. Therefore, the details disclosed herein are not to be interpreted as limiting, but merely as the basis for the claims and for teaching one skilled in the art of the invention. The numerical data or ranges used in the specification are not to be construed as limiting. The following detailed description of the preferred embodiments will now be described in accordance with the attached drawings.

The present invention relates to antimicrobial elastomeric articles, wherein the antimicrobial elastomeric articles having antimicrobial coating. The antimicrobial coating is prepared from antimicrobial coagulant solution which comprises antimicrobial additive derived from plant. The antimicrobial elastomeric article is an antimicrobial glove.

For the purpose of the present invention, the term "coating" is related to a formation of a layer of film on a glove former at multiple stages during the manufacturing process to yield a finished glove product. The finished glove product is formed from more than one layer of coating, for instance coagulant coating, latex coating and so on.

First embodiment of the present invention relates to an antimicrobial coagulant solution wherein the antimicrobial coagulant solution is a mixture of an antimicrobial additive and a coagulant solution.

The antimicrobial additive is derived from plant, wherein the antimicrobial additive is plant extract which is either a neem extract or a lemon extract. Hereinafter, the present invention is based on two antimicrobial coagulant solutions which are as below:
(i) neem extract based coagulant solution; and
(ii) lemon extract based coagulant solution.

For the purpose of the present invention, the term "antimicrobial additive" signifies the antimicrobial property exhibited by plant extract which is able to kill or suppress the growth of any microorganisms.

The plant extract is prepared by dissolving the plant extract powder in a solvent to produce a solution with undissolved material. Thereafter, the solution with undissolved material is filtered to yield an extract solution which is either neem extract solution or lemon extract solution. The solvent being used to obtain neem extract solution is any one selected from a group consisting of distilled water, tap water, deionized water and any combinations thereof, preferably distilled water. The solvent being used to obtain lemon extract solution is any one selected from a group consisting of ethanol, methanol and any combinations thereof, preferably ethanol. The undissolved material is the leftover plant extract powder that has not dissolved.

Table 1 shows the formulation of the neem extract solution.

**Table 1: Formulation of the neem extract solution**

| **Preferred chemical** | **Alternative chemical** | **Working range (%)** | **Preferred range (%)** | **Typical value (%)** |
|---|---|---|---|---|
| Neem extract powder (w/v) | Indian pennywort extract | 20.0 to 50.0 | 26.0 to 35.0 | 28.50 |
| Distilled water (v/v) | Tap water or deionized water | 50.0 to 80.0 | 65.0 to 74.0 | 71.50 |

Thereafter, the neem extract solution is mixed with coagulant solution to produce the neem extract based coagulant solution. The neem extract solution is used in an amount ranging between 0.40% to 1.00% by w/v, preferably between 0.60% to 0.80% by w/v, still preferably 0.70% by w/v in the neem extract based coagulant solution.

Table 2 shows formulation of the lemon extract solution.

**Table 2: Formulation of the lemon extract solution**

| **Preferred chemical** | **Alternative chemical** | **Working range (%)** | **Preferred range (%)** | **Typical value (%)** |
|---|---|---|---|---|
| Lemon extract powder (w/v) | Orange extract | 1.0 to 20.0 | 4.00 to 11.0 | 6.00 |
| Ethanol (v/v) | Methanol | 80.0 to 99.0 | 89.0 to 96.0 | 94.0 |

Thereafter, the lemon extract solution is mixed with the coagulant solution to produce the lemon extract based coagulant solution. The lemon extract solution is used in an amount ranging between 1.00% to 1.80% by w/v, preferably between 1.30% to 1.60% by w/v, still preferably 1.50% by w/v in the lemon extract based coagulant solution.

The coagulant solution comprises an antitack agent, a coagulating agent, a wetting agent, a pH adjuster and a solvent.

The antitack agent is any one selected from the group consisting of potassium stearate, calcium stearate and any combinations thereof, preferably potassium stearate. The antitack agent is used in an amount ranging between 0.50% to 2.00% by weight of the coagulant solution, preferably between 0.80% to 1.30% by weight of the coagulant solution, still preferably 1.00% by weight of the coagulant solution.

The coagulating agent is any one selected from the group consisting of calcium nitrate, calcium chloride and any combinations thereof, preferably calcium nitrate. The coagulating agent is used in an amount ranging between 7.50% to 8.50% by weight of the coagulant solution, preferably between 7.80% to 8.20% by weight of the coagulant solution, still preferably 8.00% by weight of the coagulant solution.

The wetting agent is any one selected from the group consisting of isotridecanol ethoxylates, dihexylsulfosuccinate and any combinations thereof, preferably isotridecanol ethoxylates. The wetting agent is used in an amount ranging between 0.01% to 0.10% by weight of the coagulant solution, preferably between 0.03% to 0.08% by weight of the coagulant solution, still preferably 0.05% by weight of the coagulant solution.

The pH adjuster is any one selected from the group consisting of ammonia, potassium hydroxide, sodium hydroxide, magnesium hydroxide, ammonium bicarbonate, ammonium chloride and any combinations thereof, preferably ammonia. The pH adjuster is used in an amount ranging between 0.001% to 0.010% by weight of the coagulant solution, preferably between 0.003% to 0.007% by weight of the coagulant solution, still preferably 0.005% by weight of the coagulant solution.

The solvent is any one selected from the group consisting of tap water, distilled water, deionized water and any combinations thereof, preferably tap water. The solvent is used in an amount to achieve 100% by weight of the coagulant solution.

Table 3 shows the formulation of the coagulant solution.

**Table 3: Formulation of the coagulant solution**

| **Ingredients** | **Working range (%)** | **Preferred range (%)** | **Typical value (%)** |
|---|---|---|---|
| Antitack agent | 0.50 to 2.00 | 0.80 to 1.30 | 1.00 |
| Coagulating agent | 7.50 to 8.50 | 7.80 to 8.20 | 8.00 |
| Wetting agent | 0.01 to 0.10 | 0.03 to 0.08 | 0.05 |
| pH adjuster | 0.001 to 0.010 | 0.003 to 0.007 | 0.005 |
| Solvent | Used in an amount to achieve 100% by weight of the coagulant solution | | |

Second embodiment of the present invention discusses on the antimicrobial gloves of present invention. The antimicrobial gloves of the present invention include at least one layer of coating, wherein the coating is antimicrobial coating. The antimicrobial coating is prepared from the above-mentioned antimicrobial coagulant solutions.

The antimicrobial gloves of the present invention are prepared adopting the commonly known method in the glove manufacturing industry.

**FIGURE 1** shows a flow diagram of the method for producing antimicrobial gloves in accordance with the exemplary embodiment of the present invention.

The method to prepare the antimicrobial gloves (100) comprise the steps of:
i. applying at least one layer of antimicrobial coagulant solution (as described in the first embodiment) on a former at a temperature ranging between 55°C to 65°C for a time period ranging between 10 seconds to 15 seconds to produce an antimicrobial coating on the former (101), wherein the coating may be applied by way of dipping the former into a dipping tank containing the antimicrobial coagulant solution or spraying the antimicrobial coagulant solution onto the former, wherein the coating may be carried out offline and/or online;
ii. drying the antimicrobial coating on the former obtained in step (i) at a temperature ranging between 130°C to 140°C for a time period ranging between 2 minutes to 3 minutes (102);
iii. dipping the former obtained in step (ii) into at least one latex dipping tank containing latex formulation at a temperature ranging between 50°C to 65°C for a time period ranging between 1 minute to 2 minutes to produce a latex layer coated on the former (103) wherein the latex formulation comprises at least a base polymer, a dispersing agent, an activator, a pH adjuster, a crosslinker, an accelerator, a wetting agent and an antifoaming agent;
iv. drying the latex layer coated on the former obtained in step (iii) at a temperature ranging between 50°C to 70°C for a time period ranging between 1 minute to 2 minutes (104);
v. pre-leaching the latex layer coated on the former obtained in step (iv) with hot water at a temperature ranging between 60°C to 70°C for a time period ranging between 1 minute to 2 minutes to leach out excess chemical residues to form pre-leached latex film (105);
vi. curing the pre-leached latex film coated on the former obtained in step (v) at a temperature ranging between 120°C to 130°C for a time period ranging between 12 minutes to 14 minutes to produce cured latex film (106);
vii. post-leaching the cured latex film coated on the former obtained in step (vi) with hot water at a temperature ranging between 50°C to 60°C for a time period ranging between 1 minute to 2 minutes to leach out excess chemical residues to obtain post-leached latex film (107);
viii. drying the post-leached latex film coated on the former obtained in step (vii) at a temperature ranging between 85°C to 90°C for a time period ranging between 1 minute to 2 minutes to produce antimicrobial glove (108); and
ix. stripping the antimicrobial glove obtained in step (viii) from the former (109).

The above method discusses on preparing antimicrobial glove through online process in which the antimicrobial coagulant solution is applied during online dipping process but it is not limited thereto. The antimicrobial coagulant solution can be applied through offline process in which the antimicrobial coagulant solution is applied on a glove after online process.

The antimicrobial glove of the present invention can be used in a variety of applications such as but not limited to healthcare, cosmetic and/or biomedical. Additionally, teachings of the present invention are not limited to gloves as person skilled in the art can adopt the teachings of the present invention for any other elastomeric articles which exhibit similar elastomeric characteristics such as dental dam, finger cot and the like products.

The antimicrobial efficacy of the antimicrobial gloves of the present invention (antimicrobial glove with neem extract based coagulant coating is represented by present invention 1 and antimicrobial glove with lemon extract based coagulant coating is represented by present invention 2) are compared with non-antimicrobial control glove and conventional antimicrobial glove according to American Society for Testing and Materials (ASTM) D7907 standard test method. The antimicrobial efficacy of the gloves is studied against gram-positive *Escherichia coli* and gram-negative *Staphylococcus aureus* bacteria. The antimicrobial efficacy is measured based on ASTM D7907 standard test method. The results obtained are tabulated in Table 4 below.

**Table 4: Antimicrobial efficacy of non-antimicrobial control glove, conventional antimicrobial glove and antimicrobial gloves of the present invention**

| **Glove types** | **Bacteria** | **Average percentage of bacteria killing rate** | | | |
|---|---|---|---|---|---|
| | | **0 min** | **1 min** | **3 min** | **5 min** |
| **Non-antimicrobial control glove** | *Staphylococcus aureus* | 12.00 | 15.76 | 18.04 | 21.99 |
| | *Escherichia coli* | 7.45 | 10.09 | 15.22 | 19.06 |
| **Conventional antimicrobial glove** | *Staphylococcus aureus* | 31.25 | 40.06 | 51.17 | 62.50 |
| | *Escherichia coli* | 24.53 | 25.47 | 27.41 | 28.30 |
| **Antimicrobial glove of present invention 1** | *Staphylococcus aureus* | 54.00 | 65.00 | 66.00 | 67.00 |
| | *Escherichia coli* | 10.63 | 27.66 | 34.04 | 38.29 |
| **Antimicrobial glove of present invention 2** | *Staphylococcus aureus* | 42.76 | 71.99 | 96.99 | 98.87 |
| | *Escherichia coli* | 38.85 | 62.69 | 82.11 | 87.40 |

Based on Table 4, it is apparent that the antimicrobial gloves of the present invention are able to kill both gram-positive *Staphylococcus aureus* and gram-negative *Escherichia coli* bacteria. The antimicrobial gloves of the present invention show higher bacteria killing rate as compared to the non-antimicrobial control glove and conventional antimicrobial glove for 1 minute, 3 minutes and 5 minutes of contact time.

As a whole, the antimicrobial gloves of the present invention are able to improve the antimicrobial efficacy and comply with the ASTM D7907 standard test method. The latter proves that the antimicrobial additives of the present invention are able to tolerate with the coagulant solution to successfully yield the antimicrobial gloves of the present invention.

The terminology used herein is for the purpose of describing particular example embodiments only and is not intended to be limiting. As used herein, the singular forms "a", "an" and "the" may be intended to include the plural forms as well, unless the context clearly indicates otherwise. The terms "comprises", "comprising", "including", and "having" are inclusive and therefore specify the presence of stated features, integers, steps, operations, elements, and/or components, but do not preclude the presence or addition of one or more other features, integers, steps, operations, elements, components, and/or groups therefrom.

The method, steps, processes, and operations described herein are not to be construed as necessarily requiring their performance in the particular order discussed or illustrated, unless specifically identified as an order of performance. It is also to be understood that additional or alternative steps may be employed. The use of the expression "at least" or "at least one" suggests the use of one or more elements, as the use may be in one of the embodiments to achieve one or more of the desired objects or result.

## Claims

1. An antimicrobial coagulant solution which is a mixture of an antimicrobial additive and a coagulant solution, wherein the antimicrobial additive is selected from the group consisting of neem extract or lemon extract and wherein the coagulant solution comprises an antitack agent, a coagulating agent, a wetting agent, a pH adjuster and a solvent.

2. The antimicrobial coagulant solution as claimed in claim 1, wherein the neem extract solution is used in an amount ranging between 0.40% to 1.00% by w/v in the antimicrobial coagulant solution.

3. The antimicrobial coagulant solution as claimed in claim 1 or 2, wherein the lemon extract solution is used in an amount ranging between 1.00% to 1.80% by w/v in the antimicrobial coagulant solution.

4. The antimicrobial coagulant solution as claimed in any of claims 1 to 3, wherein the neem extract solution comprises of neem extract powder and solvent.

5. The antimicrobial coagulant solution as claimed in claim 4, wherein the neem extract powder is used in an amount ranging between 20.0% to 50.0% by w/v in the neem extract solution.

6. The antimicrobial coagulant solution as claimed in claim 4, wherein the solvent is used in an amount ranging between 50.0% to 80.0% by v/v in the neem extract solution.

7. The antimicrobial coagulant solution as claimed in any of claims 1 to 6, wherein the lemon extract solution comprises of lemon extract powder and solvent.

8. The antimicrobial coagulant solution as claimed in claim 7, wherein the lemon extract powder is used in an amount ranging between 1.0% to 20.0% by w/v in the lemon extract solution.

9. The antimicrobial coagulant solution as claimed in claim 7, wherein the solvent is used in an amount ranging between 80.0% to 99.0% by v/v in the lemon extract solution.

10. The antimicrobial coagulant solution as claimed in any of claims 1 to 9, wherein the antitack agent is any one selected from the group consisting of potassium stearate, calcium stearate and any combinations thereof, and/or wherein the antitack agent is used in an amount ranging between 0.50% to 2.00% by weight of the coagulant solution.

11. The antimicrobial coagulant solution as claimed in any of claims 1 to 10, wherein the coagulating agent is any one selected from the group consisting of calcium nitrate, calcium chloride and any combinations thereof, and/or wherein the coagulating agent is used in an amount ranging between 7.50% to 8.50% by weight of the coagulant solution.

12. The antimicrobial coagulant solution as claimed in any of claims 1 to 11, wherein the wetting agent is any one selected from the group consisting of isotridecanol ethoxylates, dihexylsulfosuccinate and any combinations thereof, and/or wherein the wetting agent is used in an amount ranging between 0.01% to 0.10% by weight of the coagulant solution.

13. The antimicrobial coagulant solution as claimed in any of claims 1 to 12, wherein the pH adjuster is any one selected from the group consisting of ammonia, potassium hydroxide, sodium hydroxide, magnesium hydroxide, ammonium bicarbonate, ammonium chloride and any combinations thereof, and/ or wherein the pH adjuster is used in an amount ranging between 0.001% to 0.010% by weight of the coagulant solution.

14. The antimicrobial coagulant solution as claimed in any of claims 1 to 13, wherein the solvent is any one selected from the group consisting of tap water, distilled water, deionized water and any combinations thereof and/or wherein the solvent is used in an amount to achieve 100% by weight of the coagulant solution.

15. An antimicrobial elastomeric article includes at least one layer of coating, wherein the coating is antimicrobial coating and wherein the antimicrobial coating is prepared from the antimicrobial coagulant solutions as claimed in claims 1 to 14, wherein the antimicrobial elastomeric article is preferably an antimicrobial glove.
